# EUROPEAN PATENT SPECIFICATION

(11) **EP 0 295 287 B1**
(45) Date of publication and mention of the grant of the patent: **11.08.1993**
(21) Application number: 88900598.9
(22) Date of filing: 28.12.1987
(51) Int. Cl.: C12N 15/12, C12N 15/18, C12N 15/81, C12P 21/02, C12R 1/19

(54) **METHOD FOR THE PRODUCTION OF PORCINE GROWTH HORMONE USING A SYNTHETIC GENE IN YEAST CELLS**
VERFAHREN ZUR HERSTELLUNG DES SCHWEINEWACHSTUMSHORMONS MITTELS EINES KUNSTGENS IN HEFEZELLEN
PROCEDE DE PRODUCTION DE L'HORMONE DE CROISSANCE DES PORCS A PARTIR D'UN GENE SYNTHETIQUE DEVELOPPE DANS DES CELLULES DE LEVURE

(30) Priority: 31.12.1986 KR 1171086
(43) Date of publication of application: 21.12.1988
(73) Proprietor: LUCKY, LTD., Seoul 150 (KR)
(72) Inventor: CHO, Joong, Myung, Seoul 133 (KR); LEE, Tae, Ho, Daejeon-shi Chungcheongnam-do 300-31 (KR); CHUNG, Hyun, Ho, Kwanak-gu Seoul 151 (KR); LEE, Yong, Beom, Daejeon-shi Chungcheongnam-do 300-01 (KR); LEE, Tae, Gyu, Seoul 132 (KR); PARK, Young, Woo, Daejeon-shi Chungcheongnam-do 300-31 (KR); HAN, Kyu, Beom, Daejeon-shi Chungcheongnam-do 300-31 (KR)
(74) Representative: Perry, Robert Edward
(86) International application number: KR8700015
(87) International publication number: WO8805080

(56) References cited:
- EP-A- 0 104 920
- EP-A- 0 111 389
- EP-A- 0 208 489

## Description

The present invention relates to a method for the production of porcine growth hormone (pGH) by a synthetic gene in yeast cells, to improve the growth rate of pigs and the efficiency of feed.

Traditionally, pig breeders have used feed with a high protein content or synthetic steroids in order to promote growth and improve the efficiency of feed. But because feed with steroid supplements is not metabolised quickly but remains in the body for a long time and has a detrimental influence on humans, developed nations are prohibiting its use.

EP-A-0111389 describes how pGH may be expressed in yeast, inter alia by steps comprising (a) synthesising oligonucleotides which are based on the amino-acid sequence of pGH; (b) cloning the pGH gene into an E. coli vector between a promoter and a terminator; (c) recloning the resultant promoter-pGH gene-terminator cassette into a yeast expression vector; and (d) expressing the resultant vector in yeast cells.

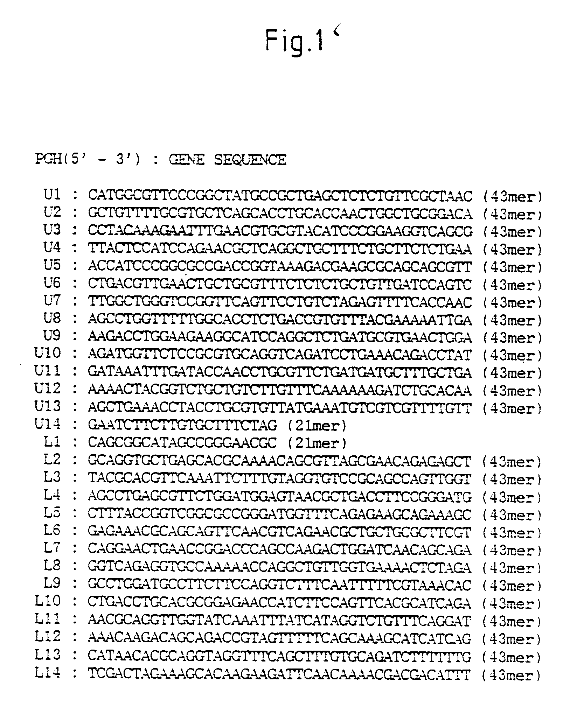

It has now been discovered that pGH may be produced more effectively if the oligonucleotides are synthesised with SacI, XbaI and SalI restriction sites and codons preferentially used in yeast, as shown in the nucleotide sequence of Figure 1, and in that the pGH gene is cloned with a N-terminal synthetic adaptor having the following base sequence, including NcoI and SacI restriction sites: 
5' - CATGGCGTTCCCGGCTATGCCGCTGAGCT - 3'
3' - CGCAAGGGCCGATACGGCGAC - 5'
NcoI site SacI site
The expression vector is the vector (pC1/1-PGH) cloned of a cassette comprising the promoter-pGH gene with an N-terminal synthetic adaptor-terminator, a complete 2 micron gene, a Leu 2d gene and an origin of replication.

In the accompanying drawings:
Fig. 1 shows synthetic oligonucleotides corresponding to the pGH gene and represented by the base sequence from the 5'-end to the 3'-end.
Fig. 2 is a ligation strategy of oligonucleotides.
Fig. 3 is a schematic cloning strategy of synthetic oligonucleotides to a vector of E.coli (pUC18).
Fig. 4 is the base sequence and putative amino acid sequence of confirmed porcine growth hormone gene.
Fig. 5 is a cloning process for a vector of E.coli(pPGAP) and a yeast expression vector (pC1/1) to produce porcine growth hormone in yeast.
Fig.6 are the results confirmed by SDS-polyacrylamide gel electrophoresis of porcine growth hormone produced in yeast cells.

### DETAILED DESCRIPTION OF A PREFERRED EMBODIMENT

The present inventors have selected the base sequence of porcine growth hormone gene with an amino acid codon preferentially used in yeast cells, used upon the amino acid sequence of porcine growth hormone reported by Seeburg et al. [DNA 2(1983) 37], and the total gene of mature porcine growth hormone in the natural state, in which the N-terminus initiates with ala, is chemically synthesized with a gene synthesizer (Applied Biosystem Model 380B, USA) according to the phosphoramidate's method.

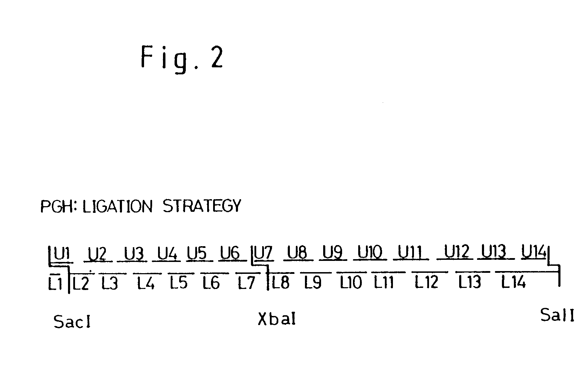

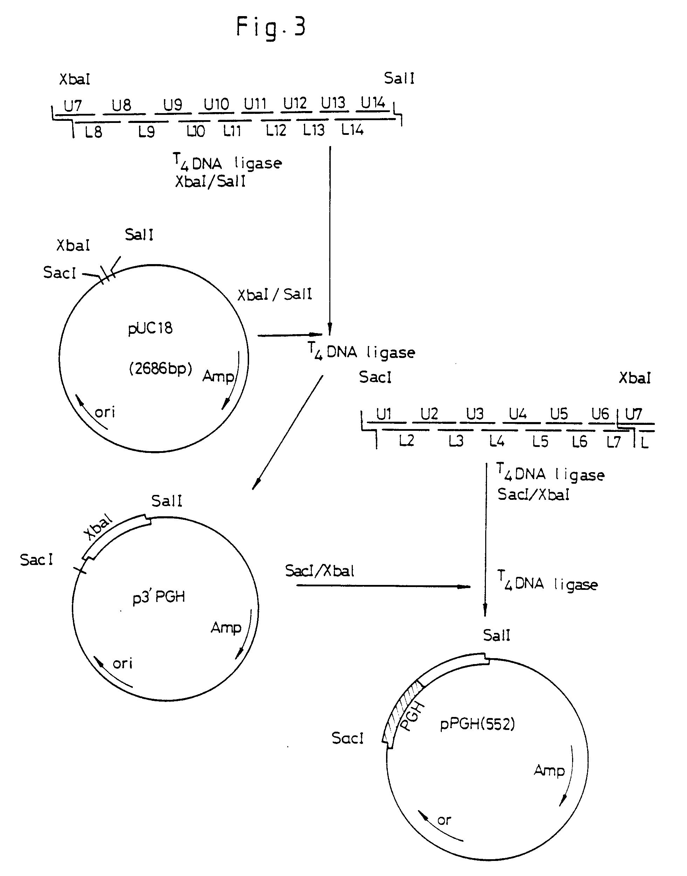

The synthetic oligonucleotides are ligated as shown in Fig. 2 and cloned to an E.coli vector, pUC18, [Norrander et al. Gene 36 (1983) 101-106] to produce a vector pPGH(552) comprising a SacI/SalI restriction fragment of 552 base pairs, which does not have an N-terminal region of a mature porcine growth hormone gene of 579 base pairs (Refer to Fig. 3).

In order to produce a clone comprising the complete porcine growth hormone gene and express it in a yeast host, the SacI/SalI fragment was separated from pPGH(552) and a synthetic adaptor was inserted between the NcoI site and the SalI site of the vector comprising a promoter and a terminator gene, pPGAP [Clontech Lab, Palo Alto, CA 94303, U.S.A. Holland, J.P. & Holland, J.J.J. of Biological Chemistry 255, 2596-2605 (1980), Bitter, G.A. & Egan, K.M. Gene 32, 263-274(1984) 20B-12, Zhu, X.L. et al., Mol. Gen. Genet 9194, 31-41(1984) ; ATCC 34025] to obtain pPGAP-PGH (579), wherein the adaptor chemically synthesized is comprised of an NcoI restriction site, an initiation codon and some amino acid codons corresponding to the N-terminal region (Refer to Example 3).

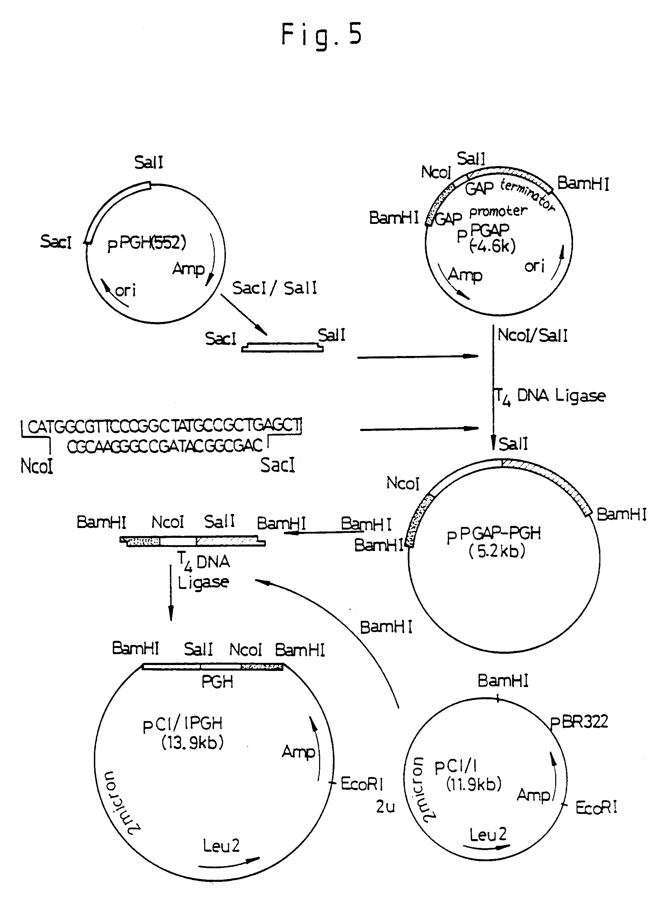

pPGAP-PGH(579) is treated with BamHI restriction enzyme to separate the BamHI fragment (about 1970 base pairs) comprising a promoter, the procine growth hormone gene and a terminator. The BamHI fragment is inserted into the BamHI restriction site of a yeast expression vector, pC1/1, which is automatically replicated in E.coli and yeast [ATCC 37115 ; Brake et al., Proc. Natl. Acad. Sci USA 81 (1984), 4642], to produce pC1/1-PGH(Refer to Fig. 5).

The expression vector is cloned to a yeast strain DCO4 [Yeast Genetic Stock Center, Broach, J.R. & Hicks, J.B. Cell 21, 501 (1980)] by means of the method of Hinnen et al. The transformed yeast cell was cultured in YEPD medium comprising 2% glucose as in sample 5 for 24 hrs, porcine growth hormone was produced depending on growth rate of the cell and 200mg of pig growth hormone per liter of culture can be obtained at the OD₆₅₀ = 20.

The invention is illustrated by the following Examples, without them limiting its range.

### Example 1 : Ligation of synthetic oligonucleotides of porcine growth hormone gene and cloning to vector pUC18

In order to obtain the complete porcine growth hormone gene from the synthetic oligonucleotides having the gene sequences of Fig. 1, ligation strategy such as in Fig. 2 and vector pUC18 comprising SacI, SalI, XbaI, etc. restriction sites were used.

The oligonucleotides (U7-U14/L8-L14) corresponding to the 3'-end of the XbaI and SalI restriction fragments from synthetic oligonucleotides were collected at the amount that the OD₂₆₀ of each oligonucleotide equals to 0.05 and then separately dried. Four units of T4 polynucleotide kinase were added to a total volume of 30 µl in the presence of a buffer solution comprising 50mM Tris-HCl(pH7.5), 1mM ATP, 1mM DTT and 10mM MgCl₂ and they were reacted at 37 °C for 30 mins. to phosphorylate the 5'-end residue of the oligonucleotides. After the oligonucleotides were pooled and treated with an equal volume of phenol and chloroform mixture, they were precipitated with ethanol. The precipitate was dissolved in 53 µl of a buffer solution comprising 60mM Tris-HCl (pH7.5), 1mM DTT and 10mM MgCl₂. The solution was placed in a 95 °C water bath and kept at room temperature for 6 hrs. so that as its temperature drops slowly, each oligonucleotide produced base pairing with complementary sequence.

T4 DNA ligase (20 units) and 5 µl of 10mM ATP were added and the 5'- and 3'-ends of the oligonucleotides were ligated at room temperature for 10mins. The above solution was treated with phenol and chloroform mixture and precipitated with ethanol.

Ten units each of XbaI and SalI restriction enzymes were added to the precipitated nucleic acid in the presence of a buffer solution comprising 60mM Tris(pH7.6), 10mM MgCl₂ and 100mM NaCl and reacted at 37 °C for 1 hr.

After 7% polyacrylamide gel electrophoresis of the above mixture, a band corresponding to 200-300 base pairs was cut from the gel. After electroelution, the precipitates were dissolved in 20 µl of distilled water.

Three µl of DNA and 10ng of a vector, pUC18, cut with XbaI and SalI restriction enzymes were ligated in the presence of the ligation solution comprising 60mM Tris-HCl(pH7.5), 10mM DTT, 10mM MgCl₂, 1mM ATP and 10units of T4 DNA ligase at 14 °C for 16hrs.

E.coli JM103[BRL, U.S.A., Messing, J., Methods in Enzymology, 103, 20-78 (1983)] competent cell was added to the ligation reactant and transformed according to Hanahan's method [J.Mol. Biol 116, 557(1983)] at 37 °C.

The clone containing p3'-PGH was selected from the white colonies by using Birnboim and Doly's method [Nucleic Acid Res. 7, 1513 (1979)].

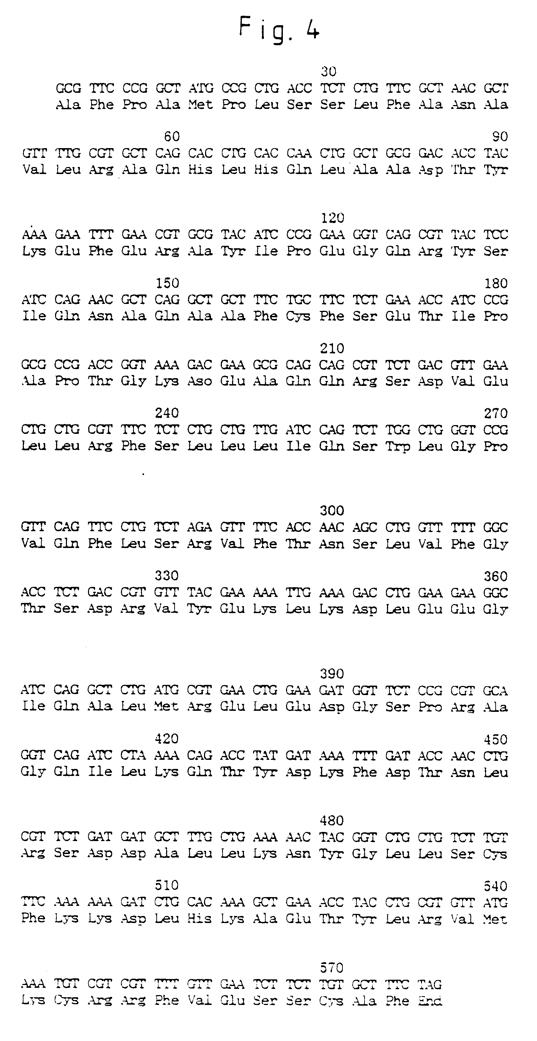

On the other hand, the oligonucleotides (U1-U7/L2-L8) corresponding to the 5'-end SacI and XbaI restriction fragment of the complete porcine growth hormone gene were ligated by the same method as mentioned above and cloned to the p3'-PGH vector cut with SacI and XbaI restriction enzymes to produce pPGH(552) as shown in Fig. 3. The nucleotide sequence was confirmed by Sanger's dideoxy sequencing method [Proc. Natl. Acad. Sci. USA 74, 5473-5477] (Refer to Fig. 4).

### Example 2 : Manipulation of synthetic porcine growth hormone gene for expression in yeast cell

pPGH(552) does not comprise 9 amino acids in the 5'-residue of a complete porcine growth hormone and in order to clone the complete porcine growth hormone gene and to have it expressed in yeast cells, the deficient part of the 5'-end was synthesized by the gene synthesizer. The adaptor comprising the NcoI restriction site, the initiation codon and the codon corresponding to 8 amino acids was synthesized by selecting codons preferentially used in yeast cells and the SacI restriction site was synthesized at the other end (Refer to Fig. 5).

The process of cloning was as follows ; pPGH(552) was treated with SacI and SalI restriction enzymes to obtain a restriction fragment corresponding to 552 base pairs and the fragment was separated through agarose gel electrophoresis. The fragment and the synthetic adaptor were inserted between the NcoI and SalI restriction sites of a vector, pPGAP, comprising a promoter and a terminator. The detailed procedures are as follows : The 5'-end of each synthetic adaptor was phosphorylated with T4 polynucleotide kinase as described in sample 1. One µl of each phosphorylation solution, 3 µl (30ng) of the SacI/SalI fragment separated from pPGH(552) and 1 µl (7ng) of vector pPGAP cut with NcoI and SalI restriction enzymes were mixed and kept at 65 °C for 15 mins. They were cooled slowly to room temperature. Two µl of 10mM ATP, 2 µl of a 10-fold concentrated ligation reaction buffer solution, 1 µl of ligase and 8 µl of distilled water were added and reacted at 14 °C for 16 hrs.

Based upon the method as in Example 1, the above was cloned to E.coli cell HB101 [ATCC 37017], and pPGAP-PGH comprising the promoter, the complete porcine growth hormone gene and the terminator for expression in yeast was produced.

The vector, pPGAP, contains a glyceraldehyde-3'-phosphate dehydrogenase promoter, a constitutive promoter which is expressed according to the growth of the cell and a terminator. pPGAP-PGH was treated with BamHI restriction enzyme to separate the BamHI restriction fragment about 1,970 base pairs, comprising a glyceraldehyde-3'-phosphate dehydrogenase promoter, a porcine growth hormone gene and a glyceraldehyde-3'-phosphate dehydrogenase terminator. The BamHI restriction fragment was inserted into the BamHI restriction site of the expression vector pC1/1 which can be replicated in yeast cells, to produce pC1/1-PGH (Refer to Fig. 5).

The pC1/1-PGH vector was transferred into yeast strain DCO4 according to Hinnen's method [Proc Natl. Acad. Sci, USA 75 (1978), 1929].

After culturing at 30 °C for 5 days, a recombinant clone with porcine growth hormone gene was picked and identified by the method such as in Example 3.

### Example 3 : Cultivation of yeast for producing porcine growth hormone and its identification

Each 3ml of yeast cells transformed with vector pC1/1-PGH was cultured in a culture medium without leucine (6.7g of Yeast Nitrogen Base without amino acids, 0.25g of leu-deficient supplements and 6% glucose per l of culture medium) at 30 °C for 24hrs. The culture was added to 100ml of YEPD culture medium comprising 2% peptone, 1% yeast extract and 2% glucose and cultured at 30 °C for 24 hrs.

At the OD₆₅₀ equaled to around 40, the fraction corresponding to OD₆₅₀ of 10 was collected and centrifuged. It is dissolved in 400 µl of a buffer solution containing 10mM Tris-HCl(pH 7.5), 1mM EDTA, 2mM phenyl methyl sulfonyl fluoride and 8M urea and glass beads, 0.45mm in diameter with the same volume, were added and shaken vigorously. After rupturing the cell wall and letting porcine growth hormone elute into the buffer solution, 4 µl of eluted solution was executed by electrophoresis on 12.5% SDS polyacrylamide gel.

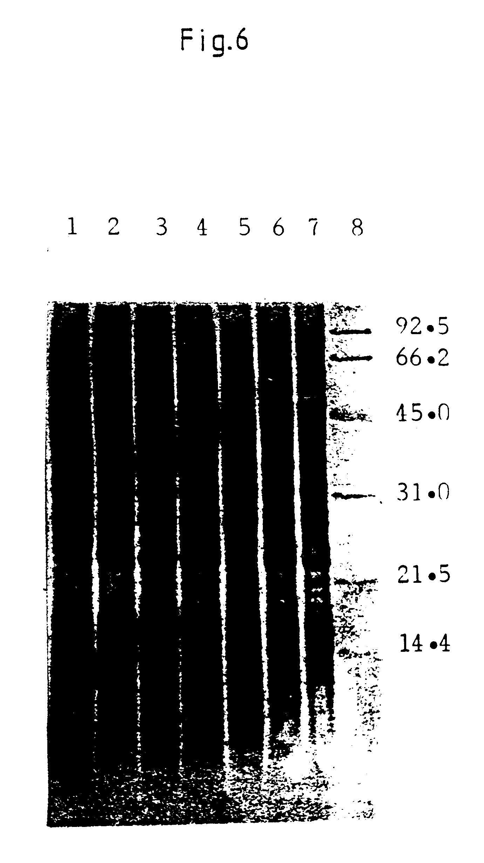

The results are represented in Fig. 6;
- Lane 8: represents the standard M.W. of protein from the BioRad company.
- Lanes 1 and 5: represent total proteins of yeast cells transformed with the vector pC1/1 without porcine growth hormone gene.
- Lanes 2-4: represent total proteins of yeast cells transformed with the vector pC1/1-PGH containing the porcine growth hormone gene.

As shown in lanes 2-4 of Fig 6, it is evident by gel scanning that porcine growth hormone appears at a hand about 22Kd in an amount corresponding to 10% of the total protein.

The amino acid sequence confirmed (Biomedical Resource Center, University of Calfornia, San Francisco) shows the mature porcine growth hormone starting from alanine and in which methionine might be processed by amino peptidase in vivo.

## Claims

1. A method for the production of porcine growth hormone (pGH), comprising:
(a) synthesizing oligonucleotides which are based on the amino-acid sequence of pGH;
(b) cloning the pGH gene into an E. coli vector between a promoter and a terminator;
(c) recloning the resultant promoter-pGH geneterminator cassette into a yeast expression vector; and
(d) expressing the resultant vector in yeast cells;
characterized in that the oligonucleotides are synthesized with SacI, XbaI and SalI restriction sites and codons preferentially used in yeast, as shown in the nucleotide sequence of Figure 1, and in that the pGH gene is cloned with a N-terminal synthetic adaptor having the following base sequence, including NcoI and SacI restriction sites:

5' - CATGGCGTTCCCGGCTATGCCGCTGAGCT - 3'
3' - CGCAAGGGCCGATACGGCGAC - 5'
NcoI site SacI site

## Patentansprüche

1. Verfahren zum Herstellen von Schweinewachstumshormon (pGH), umfassend:
(a) Synthetisieren von Oligonucleotiden, welche auf der Aminosäurensequenz von pGH basieren;
(b) Klonieren des pGH-Gens in einen E. coli-Vektor zwischen einen Promotor und einen Terminator;
(c) erneutes Klonieren der sich ergebenden Promotor-pGH-Gen-Terminator-Kassette in einen Hefeexpressionsvektor; und
(d) Exprimieren des sich ergebenden Vektors in Hefezellen;
dadurch **gekennzeichnet,** daß die Oligonucleotide mit SacI-, XbaI- und SalI-Restriktionsstellen und Codons, welche vorzugsweise in Hefe verwendet werden, wie in der Nucleotidsequenz von Fig. 1 dargestellt, synthetisiert werden, und daß das pGH-Gen mit einem N-terminalen synthetischen Adaptor mit der nachfolgenden Basensequenz, umfassend NcoI- und SacI-Restriktionstellen: 
5' - CATGGCGTTCCCGGCTATGCCGCTGAGCT - 3'
3' - CGCAAGGGCCGATACGGCGAC - 5'
NcoI-Stelle SacI-Stelle
 kloniert wird.

## Revendications

1. Méthode de production de l'hormone de croissance porcine (pGH), comprenant:
(a) la synthèse d'oligonucléotides qui sont basés sur la séquence d'aminoacides de la pGH;
(b) le clonage du gène de la pGH dans un vecteur d'E. coli entre un promoteur et un terminateur;
(c) le clonage à nouveau de la cassette promoteur-gène de pGH-terminateur obtenue dans un vecteur d'expression de levure; et
(d) l'expression du vecteur obtenu dans des cellules de levure;
caractérisée en ce que les oligonucléotides sont synthétisés avec des sites de restriction SacI, XbaI et SalI et des codons utilisés de façon préférentielle dans les levures, comme le montre la séquence de nucléotides de la figure 1, et en ce que le gène de pGH est cloné avec un adaptateur synthétique N-terminal ayant la séquence de bases suivantes, comprenant les sites de restriction NcoI et SacI:
